(19) Europäisches Patentamt
European Patent Office
Office européen des brevets

(11) Publication number: 0 018 019 A1

# (12) EUROPEAN PATENT APPLICATION

(21) Application number: **80200074.5**

(22) Date of filing: **28.01.80**

(51) Int. Cl.³: **A 01 N 59/00**, A 01 N 41/02, A 01 N 41/04, C 11 D 3/48, A 61 K 7/16

(30) Priority: **09.02.79 US 10872**

(43) Date of publication of application: **29.10.80 Bulletin 80/22**

(84) Designated Contracting States: **AT BE CH DE FR GB IT LU NL SE**

(71) Applicant: **THE PROCTER & GAMBLE COMPANY, 301 East Sixth Street, Cincinnati Ohio 45202 (US)**

(72) Inventor: **Zimmerer, Roger Earl, 8891 Woodview Drive, Cincinnati Ohio 45231 (US)**
Inventor: **Berg, Ronald Wayne, 6353 Delcrest Drive, Fairfield Ohio 45014 (US)**

(74) Representative: **Munro, Hamish David et al, PROCTER & GAMBLE EUROPEAN TECHNICAL CENTER Temselaan 100, B-1820 Strombeek-Bever (BE)**

(54) **Antimicrobial water-soluble compound which contains a rare earth metal cation, its application in oral compositions and method for controlling microorganisms therewith.**

(57) Antimicrobial compounds and compositions comprise water-soluble metallo-surfactants of the formula

$$(RX^{\ominus})_n M^{n\oplus}$$

wherein M is a rare earth metal cation; X is a sulfate or sulfonate moiety; and R is selected from various hydrocarbyl groups, hydrocarbyl aryl groups, substituted hydrocarbyl groups (such as the alkyl succinates, alkyl glycerides, alkyl glyceryl ethers and hydrocarbyl alkylene oxides) and hydrocarbyl ammonium, phosphonium and sulphonium groups. The compositions herein provide broad spectrum antimicrobial benefits and are useful in a wide variety of medicinal, cosmetic and cleansing compositions.

EP 0 018 019 A1

TITLE MODIFIED see front page

# ANTIMICROBIAL COMPOSITIONS

The desirability of controlling or eradicating common disease-causing microorganisms on, e.g., the bodies of humans and lower animals, on textiles, hard surfaces, etc., is clearly accepted. For use on humans and other animals in particular, it is highly desirable to provide compositions for controlling said microorganisms without harming the host. When the microorganisms are bacteria, it is usually desirable to use antimicrobial agents that are capable of killing or controlling both Gram-positive and Gram-negative species.

It has now been discovered that rare earth metals can be used in synergistic combination with certain surfactants to provide excellent antimicrobial agents.

A wide variety of cationic and halogenated antimicrobial agents are known in the art. However, the antimicrobial agents of the present invention offer several advantages over the art-disclosed antimicrobials. The agents herein are less toxic, orally, than the common cationic surfactant-type antimicrobials, and are less persistent in the environment than halogenated antimicrobials. In contrast with many art-disclosed antimicrobials, microorganisms do not appear to form mutant strains which are resistant to the agents of this invention. Also in contrast with many art-disclosed antimicrobials, the agents herein can be used at low concentrations to control both Gram-positive and Gram-negative bacteria. The agents herein are acceptably mild even when relatively high concentrations are used in contact with skin. Moreover, the agents of this invention kill

bacteria with only minimal contact time. Accordingly, the antimicrobial agents herein are especially useful in hard surface cleaners, cosmetics and oral compositions such as dentifrices and mouthwashes.

## Background Art

Belgian Patent 856,751 discloses cerium salts of sulfadiazine, especially cerous sulfadiazine, for treating burns. These salts are obtained from reacting water-soluble salts of cerium, say, cerium chloride or cerium nitrate, with an aqueous solution of sulfadiazine, say sodium sulfadiazine (alkali metal salt of sulfadiazine). The cerium salt of sulfadiazine is said to display antibacterial activity at low concentrations of 0.05 micro-mole/ml.

A. Yu Zimakov, et al., RARE EARTH ELEMENTS IN MEDICINE (Kazanskii Meditsinskii Zhurnal, 57(3):268-271 (1976)). This paper relates generally to the antibacterial activity of rare earth metals. More specifically, the article discusses the antibacterial activity of inorganic salts of rare earth metals, e.g., cerium nitrate at 1000 ppm inhibits Staphylococcus and molds. Reference was made to the therapeutic benefits of sulfates of neodymium, praseodymium, samarium and acetates of lanthanum in the treatment of tuberculosis.

Sudie Burkes, et al., THE BACTERIOSTATIC ACTIVITY OF CERIUM, LANTHANUM, AND THALLIUM, Journal of Bacteriology 54:417-24 (1947). This article reports the bacteriostatic properties of the captioned rare earth metals at concentrations which are said to be significantly low, ranging from 0.0006 to 0.0012 M. Inorganic salts of the captioned rare earth metals were at these concentrations and were found to have bacteriostatic properties.

Charles Levinson, et al., LANTHANUM-INDUCED ALTERATION IN CELLULAR ELECTROLYTES AND MEMBRANE POTENTIAL IN EHRLICH ASCITES TUMOR CELLS, Journal of Cellular Physiology 79:299-308 (1971). The effect of lanthanum at varying concentrations from 0.01 to 2.0 mM on membrane potential and electrolyte composition of Ehrlich ascites tumor cells was investigated. $La^{+3}$ concentrations less

than 0.02 mM had no effect. At 1.0 mM the effect was maximal and resulted in an 87% reduction in cellular potassium, 79% reduction in chloride ions and 21% reduction in sodium within 4.8 minutes. The sodium loss occurred even in the face of an electrochemical potential gradient favoring sodium entry. In short, this article shows that $La^{+3}$ affects the permeability of animal cell membranes.

Hunt, U.S. Patent 3,773,813, discloses a process for preparing anionic metal sulfonates. Lanthanum and yttrium are disclosed as some of the metal constituents. These anionic metal sulfonates are put to use as standards for spectographic equipment.

Conostan, a technical publication by the Conostan Division, (Continental Oil Company (Oklahoma)) discloses the inclusion of lanthanum anionic surfactant combinations in oil as internal standards for spectrometric analysis.

## Disclosure of Invention

The present invention is based on the discovery that combinations of rare earth metals and certain surfactants possess synergistic antimicrobial properties. (The term "synergistic" as used herein means that combinations of the two discrete entities, i.e., the rare earth metal and the surfactant, display a degree of enhanced antimicrobial activity which is far greater than that of either entity used alone.) The superior antimicrobial properties of the compositions herein are demonstrated hereafter. The data are from standard tests for measuring Minimum Bactericidal Concentrations (using a 10-minute contact with the test agent at 37°C; representing at least 3 log unit reduction in viable cell count).

Alone, inorganic rare earth salts such as the lanthanum salts do not possess appreciable antibacterial activity under the test conditions, as demonstrated by the following data.

Minimum Bactericidal Concentration

| Compound | E. coli | S. aureus |
|---|---|---|
| $LaCl_3 \cdot 6H_2O$ | >3500 ppm | >3500 ppm |
| $La(NO_3)_3 \cdot 6H_2O$ | >4300 ppm | >4300 ppm |
| $La_2(SO_4)_3 \cdot 8H_2O$ | >7100 ppm | >7100 ppm |

Certain anionic and zwitterionic surfactants are somewhat bactericidal to Gram-positive bacteria at moderate to high concentrations, but none possess appreciable activity against Gram-negatives. Lanthanum potentiates the activity of certain anionic and zwitterionic surfactants, rendering them bactericidal at low concentrations to both Gram-positive and Gram-negative bacteria, as demonstrated by the following data.

Minimum Bactericidal Concentration (ppm)

| Compound | E. coli as Na salt | E. coli Na salt + $LaCl_3$* | S. aureus as Na salt | S. aureus Na salt + $LaCl_3$* |
|---|---|---|---|---|
| Linear Alkylate Sulfonates | | | | |
| 1-$C_6$LAS | >2000 | 32 | >2000 | >500 |
| 2-$C_6$LAS | >2000 | 16 | >2000 | >500 |
| 1-$C_8$LAS | >2000 | 16 | >2000 | >500 |
| 2-$C_8$LAS | >2000 | 16 | >2000 | 125 |
| 1-$C_{10}$LAS | >2000 | 8 | 1000 | 63 |
| 2-$C_{10}$LAS | >2000 | 32 | 2000 | 32 |
| 2-$C_{12}$LAS | >2000 | 32 | 125 | 32 |
| 4-$C_{12}$LAS | >2000 | 500 | 1000 | 32 |
| 6-$C_{12}$LAS | >2000 | 500 | 125 | 250 |

* 3 molecular equivalents of Na surfactant to 1 molecular equivalent of $LaCl_3$.

As will be demonstrated more fully hereinafter, the rare earth metals can be used in combination with a wide variety of surfactants to provide superior, broad-scale antimicrobial agents.

Accordingly, the present invention encompasses water-soluble compounds especially useful as antimicrobial agents, said compounds being of the formula

$$(RX^{\ominus})_n M^{n\oplus}$$

wherein M is a rare earth metal cation; X is a sulfate or sulfonate moiety; and R is selected from hydrocarbyl groups containing from about 8 to about 18 carbon atoms, hydrocarbyl aryl groups wherein the hydrocarbyl portion contains from about 8 to about 14 carbon atoms, substituted hydrocarbyl groups wherein the hydrocarbyl portion contains from about 6 to about 18 carbon atoms, and hydrocarbyl ammonium, phosphonium or sulphonium groups of the type $R^1R^2R^3N^{\oplus}(CH_2)_x$— or $R^1R^2R^3P^{\oplus}(CH_2)_x$— or $R^1R^2S^{\oplus}(CH_2)_x$— wherein $R^1$ is $C_8$-$C_{16}$, $R^2$ and $R^3$ are each

$C_1$-$C_5$, x is an integer from about 1 to about 8, and n is 3 or 4, corresponding to the valence of the rare earth metal.

For the reasons disclosed more fully hereinafter, when group R is hydrocarbyl aryl and has an alkyl hydrocarbyl chain of about 12 or more carbon atoms, the aryl substituent is not affixed to the terminal carbon atom of the chain, but rather is affixed to any of the other carbons, preferably the number 2 or number 3 carbon atom.

The invention also encompasses synergistic antimicrobial agents comprising aqueous solutions of the rare earth metal cation and surfactants, as disclosed herein.

The invention also encompasses methods for controlling microorganisms in vitro and in vivo, comprising contacting said microorganisms with a microorganism-controlling amount of a compound or composition of the foregoing type.

Specific, but non-limiting examples of compositions employing the instant antimicrobial agents include: dentifrices, mouthwashes, oral lozenges, tooth powder, chewing gum, shampoos, hard surface cleansers and sanitizers, skin sanitizers (e.g., surgical scrubs, bar soaps), deodorants, veterinary medicaments, and the like.

All "percentages" and "parts" herein are by weight, unless otherwise specified.

The term "rare earth" as used herein includes scandium and yttrium, which are "precursors" of the rare earth family of elements.

## Best Mode

The antimicrobial compositions of the instant invention comprise:

1) a water-soluble surfactant component, and
2) a rare earth metal component.

The preparation of the antimicrobial compositions herein involves simply admixing the surfactant (preferably in the form of an inorganic salt such as the sodium, potassium or ammonium salts), water and water-soluble rare earth metal salt, e.g., rare earth metal chlorides (preferred), nitrates or sulfates thereof. Preferably the molar ratio of the surfactant to the rare earth metal salt is about 3:1, but ratios of from about 1:1 to about 10:1 (surfactant:rare earth metal salt) can optionally be used.

The following exemplifies the preparation and isolation of lanthanum 4-(1'-methylnonyl)benzenesulfonate ("LaLAS"), which is the most highly preferred antimicrobial agent of this invention.

### Example I

A solution of sodium 4-(1'-methylnonyl)benzenesulfonate, 5.0 g (0.0156 mole), in 500 mls of distilled water was prepared by stirring and heating to 40°C in a 1 liter glass beaker. While stirring this solution at 35-40°C a solution of lanthanum chloride hexahydrate, 2.025 g (0.0057 mole), in 250 mls of distilled water was added over a period of 1 minute. The mixture was stirred for an additional hour at 25-30°C and the precipitate recovered by vacuum filtration. After drying overnight at ambient conditions and 4 hours *in vacuo* over $CaSO_4$, 3.65 g of solids were obtained. Upon analysis the following values were determined: %C = 51.34, %H = 7.58, %S = 7.99, %La = 13.43, %Na = 0.21, %Cl = 0.18, $\%H_2O$ = 7.90. Calculated for $C_{48}H_{75}O_9S_3La$: %C = 55.98, %H = 7.29, %S = 9.30,

%La = 13.41. From these values the following composition was calculated: 88.5% lanthanum 4-(1'-methylnonyl)-benzenesulfonate, 3.0% sodium 4-(1'-methylnonyl)benzenesulfonate, 0.5% lanthanum chloride hexahydrate, 8.0% water.

It is to be understood that, once prepared, the rare earth/surfactant compositions herein need not be isolated from solution in order to provide their desirable antimicrobial benefits, but can be used <u>in situ</u>. The following exemplifies a highly preferred antimicrobial mouthwash composition manufactured without isolation of the rare earth/surfactant antimicrobial.

## Example II

An oral composition containing LaLAS as the antimicrobial component was prepared as follows:

Sodium 4-(1'-methylnonyl)benzenesulfonate (250.0 g) and water (3 liters) were admixed in a beaker. The ingredients were stirred and heated to 40°C-45°C until a clear solution was obtained. This solution was transferred to a clean 20 gallon stainless steel vessel. Water (37 liters) was added to the sodium 4-(1'-methylnonyl)benzenesulfonate with stirring. Sodium saccharin (10.0 g) was added to the stirred solution of sodium 4-(1'-methylnonyl)benzenesulfonate. A flavor mixture consisting of varying amounts of benzaldehyde, cassia, eucalyptol, eugenol, linalool, menthol, phenylethyl alcohol and spearmint (60.0 g) was added to the stirred solution of sodium 4-(1'-methylnonyl)benzenesulfonate and sodium saccharin.

Separately, lanthanum chloride heptahydrate (77.4 g) and water (500 ml) were mixed and stirred at room temperature until a clear solution was formed.

The solution of lanthanum chloride was added slowly to the stirred solution of sodium 4-(1'-methylnonyl)benzenesulfonate, sodium saccharin and flavor.

Water (9.5 liters) was added to the resulting solution, which was then stirred about 5 minutes, or longer, before packaging.

It is to be understood that the rare earth/surfactant compositions herein exhibit their antimicrobial properties in aqueous systems. Moreover, the compounds herein or their rare earth metal components precipitate from solution at pH's above about 8. See: Moeller and Kremers, Chem. Rev. 37:114 (1945). Moreover, highly acid (below about pH 4) solutions of the surfactants herein have antimicrobial properties in their own right. Of course, compositions intended for oral use should have a pH above about 4 to avoid damage to dental enamel. With certain species of microorganisms, especially with *Pityrosporum ovale* which is the causative microorganism for dandruff, the compositions herein are optimally active in the pH range of 4.0-4.5.

In light of the foregoing, it will be appreciated that the antimicrobial compositions herein should be manufactured and used in solution in the pH range of from about 4 to about 8.

In the practice of this invention, lanthanum has been found to be the most preferred rare earth metal. It is readily available, inexpensive and stable over the broad pH range disclosed herein.

The preferred surfactant in the practice of this invention is 4-(1'-methylnonyl)benzenesulfonate (sometimes referred to as "2-$C_{10}$LAS") which is a component of widely available commercial surfactant mixtures.

The following data show the broad spectrum antimicrobial activity of lanthanum combined with 2-$C_{10}$LAS (the overall composition being referred to simply as "LaLAS").

| Organism | Minimum Bactericidal Conc. of LaLAS |
|---|---|
| Gram-positive | |
| Staph. aureus ATCC 6538 | 32 ppm |
| Staph. epidermidis ATCC 14990 | 32 ppm |
| Micrococcus luteus ATCC 4698 | 125 ppm |
| Strep. faecalis | 32 ppm |
| Bacillus subtilis | 500 ppm |
| Gram-negative | |
| E. coli ATCC 11775 | 32 ppm |
| Ps. aeruginosa ATCC 15442 | 16 ppm |
| Kleb. pneumoniae ATCC 4352 | 125 ppm |
| Salmonella typhimurium | 125 ppm |
| Proteus vulgaris | 32 ppm |

## Industrial Applicability

The water-soluble antimicrobial compositions of the instant invention comprise a water-soluble surfactant component ($RX^-$) and a rare earth metal component ($M^{n+}$).

### The Rare Earth Metal Component

The rare earth metals useful in the compounds and compositions of the instant invention are selected from scandium, yttrium, lanthanum, cerium, praseodymium, neodymium, promethium, samarium, europium, gadolinium, terbium, dysprosium, holmium, erbium, thulium, ytterbium and lutetium. Mixtures of these metals (e.g., the so-called "didymium" salts) can also be used.

The rare earths are used herein in the form of their water-soluble salts, especially the chloride salts.

The most preferred rare earth metal used herein is lanthanum, but synergistic antimicrobials can also be prepared from the other rare earths, as demonstrated by the following data.

| Rare Earth Cation (mM) | 2-$C_{10}$LAS (mM) | Log Survivors/ml* | | | | |
|---|---|---|---|---|---|---|
| | | $DyCl_3$ | $LaCl_3$ | $NdCl_3$ | $PrCl_3$ | $YbCl_3$ |
| 0.1 | 0.3 | 0 | 0 | 0 | 0 | 0 |
| 0.05 | 0.15 | 0 | 0 | 0 | 0 | 0 |
| 0.01 | 0.03 | 0 | 0 | 1 | 0 | 0 |
| 0 | 0.3 | 6 | 6 | 6 | 6 | 6 |
| 0.1 | 0 | 3 | 5 | 3 | 3 | 2 |
| 0 | 0 | 7 | 7 | 7 | 7 | 7 |

*Approximately $10^7$ E. coli/ml were exposed to the systems shown above for 10 min. at 37°C. Survivors were enumerated by tube dilution assay.

In contrast, it is noteworthy that other polyvalent metals, such as aluminum, calcium, magnesium, iron, and indium are ineffective as components of the antimicrobial compositions tested in the foregoing manner.

## The Surfactant Component

The surfactants useful in the instant invention comprise a wide variety of anionic and zwitterionic surfactants commonly known in the art for use in laundry detergents, and the like. It is to be understood that common soaps, i.e., fatty acid carboxylates, are not useful as the surfactant component of the rare earth/surfactant compositions of this invention, possibly because carboxylate soaps form insoluble precipitates with the rare earth metals.

The following data demonstrate the antimicrobial activity of compositions comprising lanthanum as the rare earth cation and a variety of non-soap surfactants.

Minimum Bactericidal Concentration (ppm)

| Compound | E. coli as Na salt | E. coli Na salt + $LaCl_3$ | S. aureus as Na salt | S. aureus Na salt + $LaCl_3$ |
|---|---|---|---|---|
| Alkyl Sulfates (AS) | | | | |
| $C_8AS$ | >2000 | 500 | >2000 | >500 |
| $C_{10}AS$ | >2000 | 63 | >2000 | 250 |
| $C_{12}AS$ | >2000 | 125 | 500 | 32 |
| $C_{13}AS$ | >2000 | 125 | 1000 | 63 |
| Other Anionics | | | | |
| Dioctylsulfosuccinate | >2000 | 32 | 1000 | 63 |
| Coconut monoglyceride sulfonate | >2000 | 16 | >2000 | 63 |
| $C_{12.5}E_3S$* | >2000 | 32 | >2000 | 63 |
| Oleyl sulfate | >2000 | 16 | 63 | 16 |

*Average 12.5 carbon alkyl triethoxy sulfate

| Compound | E. coli as Na salt | E. coli Na salt + $LaCl_3$ | S. aureus as Na salt | S. aureus Na salt + $LaCl_3$ |
|---|---|---|---|---|
| Zwitterionics | | | | |
| $C_{12}$ammoniopropane sulfonate | >2000 | 16 | 2000 | >500 |

As can be seen from the foregoing data, a wide variety of surfactants having an anionic end-group (which combines with the rare earth cation) can be used in synergistic antimicrobial combination with rare earth metals. The general molecular structure of the surfactants herein comprises a hydrophilic sulfate or sulfonate water-solubilizing group and a hydrophobic hydrocarbyl "tail."

Typically, the hydrophobic portion of the surfactants used herein will comprise a hydrocarbyl group containing from about 8 to about 18 carbon atoms, or a hydrocarbyl aryl group wherein the hydrocarbyl portion contains from about 8 to about 14 carbon atoms, or substituted hydrocarbyl groups wherein the hydrocarbyl portion contains from about 6 to about 18 carbon atoms, or hydrocarbyl cationic groups (ammonium, phosphonium, sulphonium), preferably of the type $R^1R^2R^3N^+(CH_2)_x$ wherein $R^1$ is $C_8$-$C_{16}$, $R^2$ and $R^3$ are each $C_1$-$C_5$ and x is an integer from about 1 to about 8.

Commercially-available anionic surfactants useful herein comprise those defined as the water-soluble salts, including the alkali metal, ammonium and substituted ammonium salts of organic sulfuric reaction products having in their molecular structure a hydrophobic group containing from about 8 to about 18 carbon atoms and a water-solubilizing group selected from the group consisting of sulfonic acid and sulfuric acid ester radicals.

Important examples of the synthetic detergents which can be used in the compositions of the present invention are the following, in the form of their alkali metal (e.g., sodium and potassium) ammonium and substituted ammonium (e.g., lower alkyl ammonium) salts: alkyl sulfates, especially those obtained by sulfating the higher alcohols produced by reducing the glycerides of tallow or coconut oil; random paraffin sulfonates in which

the alkyl group contains from about 8 to about 14 carbon atoms, prepared by treating random paraffin hydrocarbons in sulfur dioxide and chlorine in the presence of light followed by treating with a base; branched or linear alkyl benzene sulfonates in which the alkyl group contains from about 8 to about 14 carbon atoms, preferably from about 10 to about 14 carbon atoms, especially those of the types described in U.S. Patents 2,220,099, 2,477,383 and 3,794,605, which are incorporated herein by reference.

Typical examples of surfactants having substituted hydrocarbyl hydrophobic groups useful herein include: the sodium alkyl glyceryl ether sulfonates, especially those ethers of the higher alcohols derived from tallow and coconut oil; coconut oil fatty acid monoglyceride sulfates and sulfonates; sulfuric acid esters of the reaction product of one mole of a higher fatty alcohol (e.g., tallow or coconut alcohols) and from about 1 to about 6, preferably about 3 moles of ethylene oxide; alkyl phenol ethylene oxide ether sulfates with about 4 units of ethylene oxide per molecule and in which the alkyl groups contain about 9 carbon atoms; the reaction product of fatty acids esterified with isethionic acid and neutralized with sodium hydroxide where, for example, the fatty acids are derived from coconut oil; fatty acid amides of the methyl taurine in which the fatty acids, for example, are derived from coconut oil; sulfonated olefins of U.S. Patent 3,332,880, incorporated herein by reference; and esters of sulphodicarboxylic acid such as described in U.S. Patents 2,176,423 and 2,028,091, which are incorporated herein by reference.

Other surfactants having an anionic water-solubilizing group which can be used herein fall within the general class of zwitterionic surfactants (ammonio, sulfonio and phosphonio surfactants). Typical examples of these classes of surfactants, along with methods of

synthesis, are disclosed in U.S. Patents 3,929,678 and 4,051,234, incorporated herein by reference.

As can be seen from the foregoing, a wide variety of surfactants interact synergistically with the rare earths to provide excellent antimicrobials. As a general proposition, and depending to some extent on the particular microorganism, the optimal, broad spectrum antimicrobial activity of the compositions herein is shown with surfactants having hydrocarbyl groups in what can be termed the "medium chain length" range. That is to say, activity begins to diminish at chain lengths below about $C_6$ (hexyl) and about $C_{12}$, or above. Again, however, this depends to some extent on the microorganism and on the particular surfactant being used to form the surfactant/-rare earth composition.

In the preferred alkyl benzene sulfonates ("Linear Alkylate Sulfonates") or sulfates used herein, the hydrocarbyl group is an alkyl substituent. The alkyl group will contain about 8 to about 14 carbon atoms. As seen from the data presented hereinabove, the position of attachment of the sulfonated benzene ring to the alkyl group can affect antimicrobial activity. For example, a $C_{10}$ alkyl benzene sulfonate having the sulfonated benzene ring attached to the number 5 carbon atom of the alkyl chain would essentially provide a surfactant having two short-chain substituents, rather than a single, relatively longer-chain substituent on the benzene, and such compounds, while active, are not optimal for use as antimicrobials. In contrast, attaching the sulfonated benzene ring to the number 2 carbon atom of a $C_{10}$ alkyl group yields 2-$C_{10}$LAS, which is a preferred surfactant herein.

Similarly, attachment of the sulfonated benzene ring at the terminal carbon of, say, a linear $C_{12}$ substituent yields an n-$C_{12}$LAS surfactant which, in combination with a rare earth, is rather water-insoluble and rather poor as an antimicrobial. The present invention

specifically encompasses the more water-soluble, antimicrobially-active rare earth/alkyl benzene sulfonate compositions.

Alkyl benzene sulfonate surfactants ("LAS") for use herein include the following (wherein the integer indicates the carbon atom on the alkyl chain to which the sulfonated benzene is attached): 1-$C_6H_{13}$LAS; 2-$C_6H_{13}$LAS; 1-$C_8H_{17}$LAS; 2-$C_8H_{17}$LAS; 1-$C_{10}H_{21}$LAS; 2-$C_{10}H_{21}$LAS; 2-$C_{12}H_{25}$LAS; 4-$C_{12}H_{25}$LAS; and 6-$C_{12}H_{25}$LAS. The 1- and 2-$C_{10}$ compounds and 2-$C_{12}$ compound yield optimal results, with the 2-$C_{10}$LAS being most preferred. LAS surfactants wherein the alkyl chain is $C_{12}$, or longer, and wherein the sulfonated benzene ring is attached to the terminal carbon atom, form oil-soluble surfactants with rare earth metals.

Another type of anionic surfactant used herein comprises the alkyl (including unsaturated alkyl) sulfates, which are also well known in the detergency arts. Such compounds used herein contain an alkyl group in the $C_8$-$C_{18}$ chain length range. Again, if the chain is too short (below about $C_8$) activity drops; if the chain is too long, solubility in combination with the rare earth (and activity) drops. Antimicrobial activity can be enhanced by inserting points of unsaturation in the alkyl chain to provide alkenyl sulfates. For example, oleyl sulfate, when combined with lanthanum, shows excellent antimicrobial activity.

Typical examples of alkyl (and alkenyl) sulfate surfactants useful herein include n-$C_8H_{17}$AS; n-$C_{10}H_{21}$AS; n-$C_{12}H_{25}$AS and n-$C_{13}H_{27}$AS. As can be seen from the data presented hereinabove, the LAS surfactants are preferred over the AS surfactants for preparing rare earth/surfactant antimicrobials.

Various other types of surfactants having sulfate or sulfonate anionic hydrophobic groups and substituted

hydrocarbyl hydrophobic groups are well known in the detergency arts, as noted hereinabove. In general, the substituents on the hydrocarbyl chain serve to enhance the water solubility of such surfactants and, thus, desirably enhance the water solubility of compositions comprising such surfactants and rare earth metals. Accordingly, the hydrocarbyl group can be somewhat longer for the "substituted" surfactants than with the "unsubstituted" LAS and AS types.

Typical, non-limiting examples of materials falling within the general class of substituted surfactants useful herein include the alkyl sulfosuccinates (esters of sulfo dicarboxylic acids), the alkyl monoglyceride sulfonates, the alkyl glyceryl ether sulfonates, and the alkyl ethoxylated and alkyl phenyl ethoxylated sulfates and sulfonates with 3-30 ethoxy groups. In such compounds, the hydrophobic portion of the surfactant can contain 6 to about 18 carbon atoms, preferably 8 to about 16 carbon atoms.

The sulfonated zwitterionics are another well-known type of surfactant which can be used herein.

Specific examples of substituted and zwitterionic surfactants useful herein include: dioctylsulfosuccinate; di-coconutsulfoadipate; dioctylsulfosebacate; $C_{12.5}$ triethoxy sulfate; $C_9H_{19}$ phenyl hexaethoxy sulfate; $C_{14}$ triethoxy sulfate; $C_{16}$ pentaethoxy sulfate; coconutalkyl ethoxy (avg. 7) sulfate; coconut monoglyceride sulfonate; coconut diglycerylether sulfonate; 5-(S-3-hydroxypropyl-S-dodecylsulfonio)-3-hydroxypentane-1-sulfate; 3-(N,N-dimethyl-N-decylammonio)propane-1-sulfonate; 3-(N,N-dimethyl-N-hexadecylammonio)-2-hydroxypropane-1-sulfonate; 5-[N,N-di(3-hydroxypropyl)-N-decyldecylammonio]2-hydroxypentane-1-sulfate; and $C_{12}H_{25}N^{\oplus}(CH_3)_2(CH_2)_3SO_3^{\ominus}$.

As can be seen from the foregoing discussion and data, the antimicrobials provided by the present invention can be used in a wide variety of products.

Oral products comprising the herein-described antimicrobial compositions and an orally-acceptable carrier are one class of products encompassed by this invention. Such oral products include mouthwashes, dentifrices (toothpastes, tooth powders), chewing gum, lozenges, and the like.

Mouthwashes prepared according to this invention comprise (a) a safe and effective amount of the aforedescribed antimicrobial; and (b) an orally-acceptable carrier. The mouthwash carrier can be water or an organic solvent such as various alcohols, or mixtures thereof. Mouthwashes generally comprise a water/ethyl alcohol solution and optionally contain other ingredients such as standard flavors, sweeteners, humectants, and the like, as disclosed in U.S. Patent 4,067,962 (issued January 10, 1978) incorporated herein by reference. The alcohol helps solubilize the rare earth/surfactant antimicrobial, controls product sudsing, etc.

Mouthwashes according to this invention contain about 0.01% to 5.0% of the rare earth/surfactant antimicrobial; 0% to 40% ethyl alcohol; 0% to 20% (preferably 5% to 20%) glycerine or other humectant; 0% to 2% (preferably 0.1% to 2%) sudsing agent; 0% to 0.5% (preferably 0.05% to 0.5%) sweetening agent such as saccharin; and 0% to 0.3% (preferably 0.05% to 0.3%) flavoring agent; and the balance, water.

<u>Example III</u>

The efficacy of the antimicrobial composition LaLAS was compared with the known anti-plaque agent chlorhexidine digluconate for the prevention of dental plaque, as follows:

Lanthanum 4-(1'-methylnonyl)benzenesulfonate (LaLAS) was used in this test to reduce the *in vitro*

growth of dental plaque on teeth. This was done using a system of glass growth chambers containing clean, extracted, sound human teeth mounted in stainless steel brackets. After sterilization in an autoclave, the growth chambers were inoculated with homogenized human plaque scrapings and serially treated with Trypticase® soy broth, sucrose, artificial saliva, and solutions of plaque-inhibiting materials for four days. The plaque-inhibiting solutions were introduced for one minute each morning and afternoon. After this treatment, the teeth were withdrawn from the growth chambers, stained with an alcoholic solution of basic fuchsin to disclose any plaque that had formed and, after randomization, graded for percent plaque coverage by three trained judges. Percent plaque reduction was calculated by comparison with water-treated controls.

| Compound | % Plaque Reduction in Comparison With Water Control |
|---|---|
| Water | 0 |
| 2140 ppm LaLAS in 20% EtOH | 82 |
| 2000 ppm chlorhexidine digluconate | 84 |

In the foregoing test, the anti-plaque efficacy of LaLAS was equivalent to chlorhexidine digluconate. However, chlorhexidine digluconate undesirably promotes stains while LaLAS does not, as demonstrated by the following stain test.

Plastic artificial molars were mounted in the caps of disposable plastic vials using dental cement. A thin, organic pellicle was developed on the teeth by exposure for about 17 hours to pooled human saliva which has been centrifuged for 2 minutes at 500X gravity to remove food and similar debris. The coated teeth were serially exposed for 5 minutes to a solution of the test material, a distilled water rinse, for 5 minutes to a solution of

instant coffee and tea (20 grams of commercial instant coffee and 2 grams of commercial instant tea in 1 liter of distilled water), a distilled water rinse, the test solution for 5 minutes, a distilled water rinse, the coffee/tea solution for 5 minutes, and a final distilled water rinse. The teeth were then graded for degree of staining on a 0-8 scale (where 0 was no stain and 8 was continuous dark brown stain) by comparison with a photographic standard.

Test results were as follows:

| Test Solution | Stain Grade |
|---|---|
| $H_2O$ Control | 0 |
| 2000 ppm Cetylpyridinium chloride | 2.7 |
| 2000 ppm Chlorhexidine | 3.2 |
| 2000 ppm LaLAS | 0.1 |
| 2000 ppm LaLAS in 20% EtOH | 0 |

## Example IV

A mouthwash in accordance with the present invention is formulated as follows:

| Component | Parts by Weight |
|---|---|
| Ethyl alcohol (95% in water) | 12.00 |
| Polyoxyethylene (20) sorbitan monooleate | 0.12 |
| Sodium hydroxide (10% in water) | to pH 6.5-7.0 |
| Sodium saccharin | 0.055 |
| Flavoring | 0.16 |
| LaLAS | 0.20 |
| Color | 0.05 |
| Sorbitol (70% in water) | 12.00 |
| Distilled water | balance to 100 |

When used in the normal manner to rinse the mouth, this product is effective in retarding the formation of

dental plaque and produces appreciably lower levels of stain on the teeth than does the antiplaque agent chlorhexidine.

Example V

The mouthwash composition of Example IV is formulated by replacing the LaLAS with an equivalent amount of Ce 2-$C_{12}$LAS, Pr 4-$C_{12}$LAS, Y 2-$C_{10}$LAS, La dioctylsulfosuccinate, Nd $C_{12}$ dimethylammoniopropanesulfonate and Gd oleyl sulfate, respectively, and equivalent results are secured.

Dentifrices prepared according to the instant invention contain the instant antimicrobial, as well as various abrasive polishing materials, sudsing agents, flavoring and sweetening agents, humectants, binders and water. See also U.S. Patent 4,067,962 for disclosures of typical materials and methods for preparing dentifrices.

Certain ingredients used in many commercial dentifrice compositions are preferably avoided in dentifrices containing the rare earth/surfactant antimicrobials. For example, soluble fluorides react with the rare earths and precipitate as the metal fluorides. Certain phosphate dentifrice abrasives are somewhat soluble and can undesirably cause precipitation of rare earth phosphates.

Various inert dentifrice abrasives useful herein are known, including, by way of example, the condensation products of urea and formaldehyde as disclosed by Cooley, et al. in U.S. Patent 3,070,510 (issued December 25, 1962); silica xerogels such as those disclosed in U.S. Patent 3,538,230 to Pader, et al. (issued November 3, 1970); and mineral abrasives coated with cationic polymers such as those disclosed in U.S. patent application Serial No. 471,941, Benedict, filed May 21, 1974, incorporated herein by reference.

Example VI

A toothpaste is prepared according to the following formula:

| Component | Parts by Weight |
|---|---|
| Sorbitol (70% soln.) | 20.00 |
| Sodium saccharin | 0.21 |
| Veegum (colloidal magnesium aluminum silicate) | 0.40 |
| Precipitated urea/formaldehyde condensate (abrasive) | 30.00 |
| Flavor | 1.00 |
| Locust bean gum | 1.30 |
| Glycerine | 10.00 |
| LaLAS | 0.70 |
| Sodium lauryl sulfate | 1.50 |
| Distilled water | balance to 100 |

The toothpaste of Example VI, when used in the normal manner, is effective in retarding the formation of dental plaque and produces an appreciably lower level of stain on the teeth than does chlorhexidine.

Example VII

The toothpaste of Example VI is modified by replacing the LaLAS with 1.0 pbw of the following rare earth/surfactant compositions, respectively, and equivalent results are secured: didymium 2-$C_8$LAS, didymium 1-$C_{10}$LAS, didymium 2-$C_{12}$LAS, didymium 4-$C_{12}$LAS and didymium 6-$C_{12}$LAS.

Example VIII

A chewing gum in accordance with the present invention is formulated as follows:

| Component | | Parts by Weight |
|---|---|---|
| Gum Base | | 21.30 |
| Ester Gum | 6.40 | |
| Coumarone resin | 9.60 | |
| Dry latex rubber | 3.20 | |
| Paraffin wax (M.P. 180°F) | 2.10 | |
| Sugar | | 58.45 |
| Corn Syrup (Baumé 45) | | 18.20 |
| Flavoring | | 1.05 |
| LaLAS | | 1.00 |

Chewing this gum in the normal manner retards the formation of dental plaque and produces no appreciable staining of the teeth as does chlorhexidine.

Example IX

A shampoo composition containing LaLAS is prepared by blending the following components:

| Component | Parts by Weight |
|---|---|
| Sodium coconut alkyl glyceryl sulfonate | 15.0 |
| Sodium stearate | 8.7 |
| Perfume | 1.0 |
| LaLAS | 3.0 |
| Citric acid | to pH 4.0-4.2 |
| Water | to 100 |

The product of Example IX is effective in retarding and/or controlling the microorganism Pityrosporum ovale responsible for dandruff on hair.

Example X

A skin moisturizing composition containing LaLAS is prepared as follows:

| Component | Parts by Weight |
|---|---|
| Amerlate WFA[a] | 0.75 |
| Amerlate W[b] | 1.00 |
| Stearic acid | 1.00 |
| Cetyl alcohol | 1.75 |
| Stearyl alcohol | 0.75 |
| "OH Lan"[c] | 0.75 |
| Arlacel 165[d] | 0.75 |
| Arlamol E[e] | 1.25 |
| 2-pyrrolidinone | 8.00 |
| Distilled water | 72.55 |
| Natrosol HH[f] | 1.00 |
| Propylene glycol | 2.50 |
| Sorbitol (70% solution in $H_2O$) | 2.50 |
| Triethanolamine | 0.65 |
| LaLAS | 2.00 |
| Titanium dioxide | 0.20 |
| Perfume and minors | to 100 |

[a]Mixture of lanolin fatty acids (Amerchol Unit of CPC International).

[b]Isopropyl ester of lanolin fatty acids (Amerchol Unit of CPC International).

[c]Hydroxylated lanolin (Amerchol Unit of CPC International).

[d]Glycerol monostearate (ICI United States, Inc.).

[e]Polyoxypropylene (15) stearyl ether (ICI United States, Inc.).

[f]Hydroxyethylcellulose (Hercules, Inc.)

When used in the normal manner, the product of Example X is free from microorganisms which tend to develop in many dermatological compositions.

Example XI

A hard surface cleaning composition containing LaLAS is prepared as follows:

| Component | Parts by Weight |
|---|---|
| 3-[N,N-dimethyl-N-coconutalkylammonio]-2-hydroxypropane-1-sulfonate | 2.0 |
| Ethoxylated (7 moles) primary coconut alcohol | 4.0 |
| Straight chain random secondary alcohol ($C_{11}$-$C_{15}$) ($C_{13}$ average) | 1.5 |
| Potassium cumene sulfonate | 4.0 |
| Potassium toluene sulfonate | 4.0 |
| LaLAS | 4.0 |
| Water | to 100 |

When used in the normal manner to scrub floors, walls, windows, doors, and the like, the product of Example XI is effective in controlling the growth of microorganisms on the hard surfaces treated therewith.

Example XII

The antimicrobial hard surface cleanser of Example XI is modified by replacing the LaLAS with 5%-7% concentrations of the following antimicrobials, respectively, and equivalent results are secured: La coconut monoglyceryl ether sulfonate, Dy $C_{10}$ diethylammoniopropane sulfonate, Lu $C_{10}$AS, Nd $C_{12}$AS, Eu 2-$C_{10}$LAS, Ho 2-$C_{10}$LAS, Er 2-$C_{10}$LAS and Sm 2-$C_{10}$LAS.

Example XIII

A stable, antimicrobial solution useful in situations where sterile solutions are required, e.g., for irrigating wounds and surgical incisions, is as follows:

| Component | Parts per Million |
|---|---|
| $LaCl_3 \cdot 6H_2O$ | 45 |
| $2\text{-}C_{10}LAS$ | 135 |
| Pyrogen-free water | Balance |

As can be seen from the data presented herein and the foregoing examples, the present invention provides an excellent method for controlling microorganisms, especially in water-containing systems at a pH in the range of 4 to about 8. Aqueous compositions containing the rare earth/surfactant compositions of this invention safely provide broad spectrum antimicrobials at usage levels in the range of ca. 8 ppm, and above, preferably ca. 25-30 ppm, and above, depending on the microorganism.

Thus, the antimicrobial compositions herein are useful in a wide variety of situations where sanitization of environmental or body surfaces is desirable. Skin sanitizers comprising safe and antimicrobially-effective amounts of the rare earth/surfactant compositions and a cosmetically-acceptable cleansing-type carrier at a pH within the range from above about 4.5 to about pH 7, and shampoo compositions comprising a safe and antimicrobially-effective amount of said compositions in an acceptable shampoo-type carrier having a pH within the range of about 4 to about 4.5, are typical of the uses of the present compositions. Likewise, hard surface sanitizing cleansers comprising the present compositions and hard surface cleanser-type carriers are another use. Of course, any of these types of products are equally appropriate for both human and veterinary use, and any of

the products exemplified hereinabove can be used with pets and domestic animals of all types.

CLAIMS

1. A water-soluble compound of the formula

$$(RX^{\ominus})_n M^{n\oplus}$$

wherein M is a rare earth metal cation ; X is a sulfate or sulfonate moiety ; n is an integer which is 3 or 4, corresponding to the valence state of the rare earth metal ; and R is selected from hydrocarbyl groups containing from 8 to 18 carbon atoms, hydrocarbyl aryl groups wherein the hydrocarbyl portion contains from 8 to 14 carbon atoms provided that when said hydrocarbyl portion contains 12 or more carbon atoms the aryl portion is affixed to a carbon atom other than the terminal one of said $C_{12}$ or longer hydrocarbyl portion, substituted hydrocarbyl groups wherein the hydrocarbyl portion contains from 6 to 18 carbon atoms, and hydrocarbyl ammonium, phosphonium or sulphonium groups wherein the hydrocarbyl portion is in the $C_8$-$C_{16}$ range.

2. A compound according to Claim 1 wherein R is selected from $C_8$-$C_{14}$ alkyl, oleyl and $C_8$-$C_{14}$ alkyl benzene.

3. A compound according to Claim 2 wherein R is 4-(1'-methylnonyl)benzene.

4. A compound according to Claim 1 wherein R is selected from $C_8$-$C_{14}$ alkyl glycerol, $C_8$-$C_{14}$ alkyl glyceryl ether and $C_8$ to $C_{14}$ alkyl succinate.

5. A compound according to Claim 1 wherein R is alkyl or alkyl phenyl ethoxylate, wherein the alkyl substituent contains from 8 to 18 carbon atoms and wherein the degree of ethoxylation is from 3 to 30.

6. A compound according to Claim 1 wherein R is a dimethyl $C_8$-$C_{16}$ alkyl ammonio alkanate, wherein the alkanate substituent is $C_3$ to $C_5$ alkylene.

7. A compound according to any one of Claims 1-6

wherein M is selected from lanthanum, cerium, praseodymium, neodymium, erbium and ytterbium.

8. An oral composition, comprising :

(a) a safe and antimicrobially-effective amount of the composition of any one of Claims 1-7 as an antimicrobial agent ; and

(b) an orally-acceptable carrier

9. A composition according to Claim 8 having a pH within the range of from 4.5 to 8.

10. A method for controlling microorganisms, comprising contacting said microorganisms with an effective amount of a compound or composition according to any one of Claims 1-9.

0018019

European Patent Office

# EUROPEAN SEARCH REPORT

Application number
EP 80 20 0074

| Category | Citation of document with indication, where appropriate, of relevant passages | Relevant to claim | CLASSIFICATION OF THE APPLICATION (Int. Cl. 3) |
|---|---|---|---|
| | FR - A - 2 291 740 (WELLA)<br>* Page 3, lines 29-30; page 4, lines 3-22; examples 2,3,8 *<br>-- | 1-7,9, 10 | A 01 N 59/00<br>41/02<br>41/04<br>C 11 D 3/48<br>A 61 K 7/16 |
| | CHEMICAL ABSTRACTS, vol. 90, no. 24, June 1979, page 354, no. 192400h<br>Columbus, Ohio, U.S.A.<br>& JP - A - 79 17137 (IMPERIAL CHEMICAL INDUSTRIES LTD.) 08-02-1979<br>* Abstract *<br>-- | 8 | TECHNICAL FIELDS SEARCHED (Int.Cl. 3)<br>A 01 N 59/00<br>41/02<br>41/04<br>C 11 D 3/48 |
| A | US - A - 4 083 860 (E. RUF)<br>* Column 14, claim 1 *<br>---- | | |

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

X The present search report has been drawn up for all claims

| Place of search | Date of completion of the search | Examiner |
|---|---|---|
| The Hague | 17-07-1980 | PELTRE |

EPO Form 1503.1 06.78